# EUROPEAN PATENT APPLICATION

(11) **EP 1 813 256 A1**
(43) Date of publication of application: **01.08.2007**
(21) Application number: 05027156.8
(22) Date of filing: 13.12.2005
(51) Int. Cl.: A61K 8/37, A61K 8/23, A61Q 5/08

(54) **Bleaching/highlighting composition for hair**

(71) Applicant: KPSS-Kao Professional Salon Services GmbH, 64297 Darmstadt (DE)
(72) Inventor: Blumenschein, Katrin, 64753 Brombachtal-Hembach (DE); Uellner, Martin, 64295 Darmstadt (DE)

(57) **Abstract**

The present invention relates to water free bleaching composition in form of a dust free powder or a suspension, which comprises at least one compound with a bleaching and/or highlighting effect and a dialkyl carbonate of the general formula

R₁ O C(O) O R₂

where R₁ and R₂ are independent from each other linear or branched, saturated alkyl chains with 6 to 22 C atoms and shows excellent bleaching and/or highlighting effects.

## Description

The present invention relates to water free bleaching composition in form of a dust free powder or a granulate or a suspension, which comprises at least one compound with a bleaching and/or highlighting effect and a dialkyl carbonate and shows excellent bleaching and/or highlighting effects.

Hair bleaching is a common practice for ages. It is based on oxidative decomposition of hair colour, which is usually done using peroxide or peroxide releasing compounds such as persulfates. Due to highly irritating potential of these bleaching ingredients and dustiness of powder compositions, it is preferred to provide granular composition where dust is reduced by agglomerating small particles Into granulates using various binding agents. Most popular binding agent is mineral oil, which was the subject matter of EP 560 088 B1, Furthermore, EP 778 020 A1 suggests the use of oil and wax compounds or their mixtures for preparation of suspensions.

The bleaching of human hair customarily consists of a process with the following steps: Homogenous mixing of a water-free preparation, preferably a powder, comprising at least one compound with a bleaching or brightening effect, in particular a solid peroxide salt, preferably ammonium, potassium and/or sodium persulfate or earth alkali peroxide, with an aqueous hydrogen peroxide solution, application of this composition onto the hair, and rinsing after bleaching is completed. It has been known for some time that use of those components is effective with regard to the bleaching, but higher concentrations can lead to hair and/or scalp damage.

The present invention aims at providing a water free composition for bleaching and/or highlighting hair, which overcomes above-mentioned problems and leaves hair In improved conditions in terms of dry combability, texture, volume and shine, in addition to mildly and effectively bleaching and /or highlighting hair. The compositions of the present invention are in the form of dust free powder or suspension with relatively high consistency like a paste.

Accordingly, the object of the present invention is a water free bleaching and/or highlighting composition for keratin fibres especially for human hair comprising at least one compound with bleaching and/or highlighting effect and at least one dialkyl carbonate of the formula

R₁ O C(O) O R₂

where R₁ and R₂ are independent from each other linear or branched saturated alkyl chains with 6 to 22 C atoms.

Further objective of the present invention is a process for bleaching and/or highlighting hair wherein a water free composition comprising at least one compound with bleaching and/or highlighting effect and at least one dialkyl carbonate of the formula

R₁ O C(O) O R₂

where R₁ and R₂ are independent from each other linear or branched saturated alkyl chains with 6 to 22 C atoms is mixed with a composition comprising at least one oxidizing agent prior to application and applied onto hair and after processing 5 to 45 min rinsed of from hair and hair is optionally shampooed.

Further objective of the present invention is a process for producing bleaching and/or highlighting composition for keratin fibres especially human hair wherein powder components of water free composition is mixed in a suitable mixer and subsequently at least one dialkyl carbonate according to the general formula of above is added portion wise until either dust free granulate (agglomerates) or a suspension is obtained.

With the term water free it is meant that no additional water or any aqueous composition is added to the composition. However it should be understood that the raw materials used may comprise water low concentrations and therefore from the experience up to 1 % water content does not have any influence on the stability and incorporated into compositions of the present invention in the form of bound water of individual chemicals.

The compositions of the present invention comprises at least one dialkyl carbonate according to the general structure presented above at a concentration range of 3 to 50%, preferably 5 to 40% more preferably 7.5 to 35% and most preferably 10 to 30% by weight calculated to total composition prior to mixing with oxidizing lotion. Among dialkyl carbonates of the above formula, the most preferred ones are dicaprylyl carbonate known with the trade name Cetiol CC from Cognis and di(ethylhexyl) carbonate known with the trade name Tegosoft DEC from Degussa.

According to the present invention, the composition comprises at least one compound with bleaching and/or highlighting effect. Suitable compounds are in general peroxides. Useful as such are in particular persulfates such as sodium and potassium persulfate, ammonium persulfate, earth alkali peroxides such as magnesium peroxide, melamine peroxide or urea peroxide or phtholimidoperoxy- hexanoic acid. The proportion of peroxides is at least 5%, preferably in the range of 20 to 80% by weight, calculated to total composition prior to mixing with oxidizing lotion.

According to the invention, the water free composition can also comprise 0.1% to 10% by weight, calculated to total composition prior to mixing with oxidizing lotion, one or more ammonium salts. Suitable ammonium salts are ammonium carbonate, ammonium hydrogen carbonate, ammonium carbamate, ammonium chloride, ammonium sulfate, ammonium phosphates, ammonium nitrate, ammonium bromide, ammonium iodide, ammonium thiosulfate, ammonium molybdate, ammonium vanadate, ammonium sulfamate, ammonium citrate, ammonium salicylate, ammonium valerate, ammonium tartarate, ammonium benzoate, ammonium acetate, ammonium formiate and ammonium lactate.

Preferred thereof are the ammonium phosphates, such as NH₄H₂PO₄, (NH₄)₂HPO₄, (NH₄)₂NaPO₄, NaNH₄HPO₄ or NH₄Na₂PO₄, ammonium chloride, ammonium sulfate and diammonium hydrogen citrate, as well as ammonium chloride, preferably in an amount from 0.1% to 10% by weight, calculated to total composition prior to mixing with oxidizing lotion.

As known from EP 609 796 A2, the ammonium compounds can also be used as sole bleaching agent in respectively higher amounts.

The total proportion of the compounds with bleaching and/or highlighting effect preferably ranges from 5% to 85%, preferably 20% to 80%, more preferably 25 to 70% and most preferably 30 to 60% by weight calculated to total composition prior to mixing with oxidizing lotion.

In addition to the active component, compositions also comprise the components customarily used in such compositions: In particular inert pulverulent carrier materials, these are for example, pyrogenic silicium dioxide, starch powder, etc., alkalizing agents, such as sodium metasilicate, surface-active substances, binding agents, etc.. In order to avoid repetition, reference Is made to the respective standard literature, for example, K. Schrader, "Grundlagen und Rezepturen der Kosmetika". 2nd Ed. (1988, Hüthig Buchverlag), pages 815 to 823.

Composition of the present invention may further comprise lipophilic ingredients In addition to dialkyl carbonate such as vegetable oils, for example, jojoba oil or any other; petrolatum liquid paraffins, especially paraffinum perliquidum and parafiinum subliquidum ; silicone oils; hydropobic fatty acid esters such as octyl palmitate, isocetyl palmitate, Isopropyl palmitate and octyl stearate, C₁₀- to C₃₆-fatty acid triglycerides, as well as their mixtures. In the case that the use is wished among those the most preferred ones are jojoba oil, fatty acid esters, paraffin oils, combinations of fatty acid esters and paraffin oils as well as combinations of fatty acid esters and/or paraffin oils is particularly preferred. Concentration of these lipophilic compounds are used in a total amount of about 0.1 to 20 percent by weight, preferably from 1 to 15 percent by weight, and more preferably from 2 to 10 percent by weight, calculated to total composition prior to mixing with oxidizing lotion.

Further, In another preferred form of the invention water free composition for bleaching and/or highlighting hair comprises polymers from the group consisting of cellulose polymer compounds, alginate, polysaccarides and acrylic acid polymers, preferably methyl cellulose compounds, ethyl cellulose compounds, hydroxyethylcellulose compounds, methylhydroxyethylcellulose compounds, methylhydroxypropylcellulose compounds, carboxymethyl cellulose compounds, alginic acids, sodium alginates, ammonium alginates, calcium alginates, gum arabic, guar gum or xanthan gum, or acrylic acid polymers with molecular weights from about 1,250,000 to 4,000,000, alone or in combination with each other. The polymers are used in a total amount of 0.1 to 15 %, preferably from 0.2 to 10%, and more preferably in an amount of from 0.5 to 7.5% by weight, calculated to total composition prior to mixing with oxidizing lotion.

Bleaching and/or highlighting composition can also comprise cationic polymers as conditioning and/or thickening agents. Those are cationic cellulose type polymers know as Polymer JR type from Amerchol such as Polyquatemium 10 or cationic guar gum known with trade name Jaguar from Rhône-Poulenc and chemically for example Guar hydroxypropyl trimonium chloride. Furthermore, chitosan and chitin can also be included in the compositions as cationic natural polymers.

Furthermore, it has been found suitable those cationic polymers known with their CTFA category name Polyquatemium, Typical examples of those Polyquatemium 6, Polyquaternium 7, Polyquaternium 10, Polyquaternium 11, Polyquaternium 16, Polyquaternium 22 and Polyquaternium 28, Polyquaternium 30, Polyquaternium 37, Polyquaternium 36, Polyquaternium 46. Among those the most preferred one is the Polyquaternium 11 as well known with its trade name Gafquat from ISP and as Luviquat PQ from BASF.

As well those polymers known with their CTFA category name Quatemium are suitable. Those are for example Quatemium-8, Quaternium-14, Quaternlum-15, Quaternium-18, Quaternium-22, Quaternium-24, Quaternium-26. Quatemium-27, Quatemium-30, Quaternium-33, Quaternium-53, Quaternium-60, Quaternium-61, Quaternium-72, Quatemium-78, Quaternium-80, Quatemium-81, Quatemium-81, Quatemium-82, Quatemium-83 and Quatemium-84.

Typical concentration range for any of the cationic conditioners mentioned above can be 0.1 - 7.5% by weight, preferably 0.3 - 5% by weight and more preferably 0.5 - 2.5% by weight.

The avarage particle size of the dust free bleaching powder composition according to the Invention is generally range below 1 mm, preferably below 500 µm, more preferably less than 400 µm and in particular about 25 to about 100 µm, thus ensuring excellent processing capability, i.e. miscibility with an aqueous hydrogen peroxide solution prior to application onto human hair.

The powder composition can be produced with processes such as by mixing the powdery ingredients first and subsequently adding dialkylcarbonate and/or any other liquid component and by fluidized bed method. In fluidized bed method, powder ingredients are mixed In a vessel and made flowing by inletting an air flow which may be heated (preferred when using waxy component) or carried out at room (ambient) temperature and while the powder mix freely "flowing" dialkyl carbonate is sprayed from a nozzle mounted above the powder batch. In case that there are additional liquid ingredients those may be mixed with dialkyl carbonate prior to spraying and in the case hydrophobic waxy components are present those should be melted at elevated temperature depending on the melting point prior to spraying preferably mixed with dialkyl carbonate at the melting temperature.

It should be noted that the preferred form of the composition for bleaching and/or highlighting hair is suspension.

The bleaching and/or brightening composition of the present invention Is mixed prior to application with an oxidizing lotion comprising at least one oxidizing agent. The oxidizing agents suitable are hydrogen peroxide, urea peroxide, melamin peroxide or perborate salts. The most preferred is hydrogen peroxide. Such composition comprises 2 to 12% by weight at least one oxidizing agent preferably hydrogen peroxide and is either a solution or in the form of an emulsion. The mixing ratio is very much dependent on the level of bleaching effect aimed, i.e. the level of highlighting and/or bleaching and darkness of hair before bleaching, and can be adjusted accordingly by hair dressers, however generally mixing ratio is within the range of 0.5 to 4 by weight (bleaching composition to oxidizing lotion), preferably in the range of 1 to 2.5 by weight.

The pH of the ready to use product, mixture of bleaching composition and oxidizing lotion, is in the range of 8 to 11.5, in particular between 9 and 11.

In another form of using, the compositions of the present invention can additionally be mixed with an aqueous composition comprising one or more hair direct dye selected from anionic, cationic or neutral dyes. This part is referred as dyeing composition hereafter.

Suitable anionic direct dyes In dyeing composition are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7. D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium. Among those, the most preferred anionic dyestuffs are Acid Red 52, DC Violet 2, DC Red 33, DC Orange 4, DC Red 27 and DC Yellow 10.

Suitable cationic dyes In dyeing composition are In principal those available on the market for cosmetic hair colouring applications. For this purpose, special reference is made to the PCT application WO 95/15144 of Ciba-Geigy AG. Some examples to those are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 76, Basic Violet 1, Basic Violet 2, Basic Violet 3, Basic Violet 10, Basic Violet 14, Basic Yellow 57, Basic red 51, Basic Yellow 87 and Basic Orange 33. The most preferred ones are Basic red 51, Basic Yellow 87 and Basic Orange 33 sold by CIBA.

Additionally, the dyeing compositions of the present invention may comprise neutral dyes (HC dyes), so called nitro dyes for shading purposes. Some examples to those are: HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No. 11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No,12, HC Yellow No.13, HC Yellow No.14, HC Yellow No.16, 2-Amino-6-chloro-4-nitrophenol, picramic acid, 1,2-Diamino-4-nitrobenzol, 1,4-Diamino-2-nltrobenzol, 3-Nitro-4-aminophenol, 1-Hydroxy-2-amino-3-nitrobenzoi and 2-hydroxyethylpicramic acid.

Plant dyestuffs can also be used alone or in combination with synthetic direct-acting dyestuffs, for example henna (red or black), alkanna root, laccaic acid, indigo, logwood powder, madder root and rhubarb powder, etc,

According to the invention, the dyeing composition comprises one or more direct dye at a concentration of 0.1 to 7.5% by weight calculated to the total composition prior to mixing with bleaching and oxidizing compositions mentioned above. The dyeing composition can also comprise mixture of several direct dyes i.e. an anionic, a cationic and/or a nonionic ones. In such a case the dyes may be mixed at any ration with each other, however, the ratio of cationic dyes to acidic dyes by weight is preferably in the range of 3:1 to 1:10, more preferably 2: 1 to 1:7 and further more preferably 2:1 to 1:5.

The above mention direct dyes of cationic, anionic and nonionic character can also be added into the water free bleaching and/or highlighting composition at the concentration given in the above paragraph. The direct dyes of different characters can certainly be mixed as well.

pH of the aqueous dyeing composition of the present invention varies between 5 and 12, preferably 6-11, more preferably 6.8 to 10.5, pH is adjusted to the required pH by using triethanolamine, ammonia or its salts with acids such as ammonium chloride, ammonium sulphate, ammonium carbonate, ammonium bicarbonate, ammonium nitrate, or using alkaline solutions such as sodium hydroxide, potassium hydroxide and their respective salts with the known acids.

It should be noted that pH of the mixture of bleaching and/or highlighting composition, dyeing composition and oxidizing lotion, ready to use composition, is in the range of 8 to 11.5, in particular between 9 and 11.

Aqueous dyeing composition of present invention can comprise additionally in the base formulation fatty acids with 0 to 3 ethylenic bonds and with fatty acyl chain length of 12 to 22 C atom which may be branched or linear. Concentration of the fatty acids can be in the range of 0.1 to 10%, preferably 0.1 to 7.5% and most preferably 0.2 to 5% by weight calculated to the total composition. Fatty acid examples, without limiting the choice, suitable for colouring compositions are myristic acid, palmitic acid, behenic acid, steraic acid, oleic acid, linoleic acid, The most preferred fatty acid is oleic acid.

Aqueous dyeing compositions according to the present invention can be in the form of emulsion, solution, dispersion and/or gel. Emulsion is the preferred form.

In the case that the dyeing composition is in the form of an emulsion, it comprises as an emulsion base at least one fatty alcohol or mixture of fatty alcohols with the chain length of 14 to 22 C atoms. Examples to suitable fatty alcohols, without limiting the choice, are myristyl alcohol, cetyl alcohol, stearyl alcohol, behenyl alcohol and cetostearyl alcohol. The most preferred is cetostearyl alcohol well known with its trade name Lanette O or as Lanette N in mixture with sodium cetearyl sulfate from Cognis.

The concentration of fatty alcohol(s) is in the range from 0.5 to 20%, preferably 1 to 15% by weight, calculated to total composition prior to mixing with oxidizing and bleaching and/or highlighting composition.

Dyeing compositions according to present invention comprises surfactants selected from anionic, nonionic, amphoteric (or zwiterionic) and/or cationic surfactants as emulsifier or solubilizer, Cationic surfactants are as well used as hair conditioners In the dyeing composition.

Anionic surfactants suitable within the scope of the invention are in principal known from the cleansing compositions

These are anionic surfactants of the sulfate, sulfonate, carboxylate and alkyl phosphate type, for example, the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates.

Additional anionic surfactants useful within the scope of the invention are a-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof as well as alkyl amido polyether carboxylic acids and salts thereof. Such products have been known for some time and are on the market, for example, under the trade name "AKYPO^{®}" and "AKYPO-SOFT^{®}".

Also useful are C₈-C₂₀-acyl isethionates, alone or in admixture with other anionic surfactants, as well as sulfofatty acids and the esters thereof.

Further suitable anionic surfactants are also C₈-C₂₂-acyl aminocarboxylic acids or the water-soluble salts thereof. Especially preferred is N-lauroyl glutamate, in particular as sodium salt, as well as, for example, N-lauroyl sarcosinate, N-C₁₂-C₁₈-acyl asparaginic acid, N-myristvyl sarcosinate, N-oleoyl sarcosinate, N-lauroyl methylalanine, N-lauroyl lysine and N-lauroyl aminopropyl glycine, preferably In form of the water-soluble alkali or ammonium, in particular the sodium salts thereof, preferably in admixture with the above-named anionic surfactants.

It is also possible to use mixtures of several anionic surfactants.

An overview of the anionic surfactants suitable for the present invention can furthermore be found in the monography of K. Schrader, "Grundlagen und Rezepturen der Kosmetika", 2nd Ed. (1989, Hüthig Buchverlag), pp. 595-600 and pp. 683 to 691.

Further surfactants in the dyeing compositions according to the invention are nonionic surfactants alone or in admixture with anionic surfactants. These are described as well in Schrader, l.c., on pages 600-601 and pp. 694-695. Especially suited nonionic surfactants are, for example, long-chain fatty acid mono- and dialkanolamides, such as coco fatty acid mono- or diethanolamide and myristic fatty acid mono or diethanolamide, stearic acid mono or diethanolamide. Further nonionic surfactants suited are alkyl polyglucosides with an alkyl group of 8 to 18 carbon atoms, and with 1 to 5 glucoside units. Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}", as well as fatty alcohol ethoxylates. Further nonionic surfactants preferred in the dyeing compositions according to invention are C₁₀-C₂₂-fatty alcohol ethoxylates. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16"; The average degree of ethoxylation thereby ranges between about 2.5 and about 25, preferably about 10 and about 20.

As further surfactant component, the dyeing compositions according to the invention can also contain amphoteric or zwitterionic surfactants. Useful as such are in particular the various known betaines such as alkyl betaines, fatty acid amidoalkyl betaines and sulfobetaines, for example, lauryl hydroxysulfobstaine; long-chain alkyl amino acids, such as cocoaminoacetate, cocoaminopropionate and sodium cocoamphopropionate and -acetate have also proven suitable.

Dyeing composition can contain cationic surfactants as emulsifier, solubilizer and/or conditioning ingredients according to the formula, but not limited to. 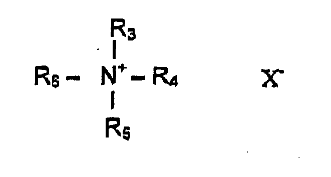 where R₃ is a saturated or unsaturated, branched or linear alkyl chain with 8-22 C atoms or

R₇ CO NH (CH₂)ₙ

where R₇ is saturated or unsaturated, branched or linear alkyl chain with 7-21 C atoms and n has typical value of 1 - 4 or

R₈ CO O (CH₂)ₙ

where R₈ is saturated or unsaturated, branched or linear alkyl chain with 7-21 C atoms and n has typical value of 1- 4, and
R₄ is H or unsaturated or saturated, branched or linear alkyl chain with 1 - 22 C atoms or

R₇ CO NH (CH₂)ₙ

or

R₈ CO O (CH₂)n

where R₇, R₈ and n are same as above.

R₅ and R₆ are H or lower alkyl chain with 1 to 4 Carbon atoms, and X is typically chloride, bromide, methosulfate.

Typical examples of those ingredients are cetyl trimethly ammonium chloride, stear trimonium chloride, dipalmitoyl dimonium chloride, distearyl dimethyl ammonium chloride, stearamidopropyl trimonuim chloride, dioleoylethyl dimethyl ammonium methosulfate, dioleoylethyl hydroxyethylmonium methosulfate.

Concentration of one or more surfactants in dyeing composition is in the range of 0.1 to 10%, preferably 0.2 to 7.5% and most preferably 0,2 - 5% by weight, calculated to the total dyeing composition.

Dyeing composition of the present invention preferably comprise an organopolysiloxane wherein at least one silicium atom is linked to an alkylene group having a hetero-atom, in particular a nitrogen atom, with a poly-(N-acyl alkyleneimine) units of the formula 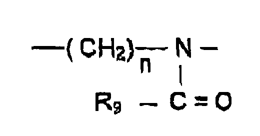 wherein n is a number from 1 to 5 and R_{g} is hydrogen, a C₁-C₁₂-alkyl or cycloalkyl, aralkyl or aryl group. Preferred organopolysiloxane polymers are those of the type disclosed in EP-A 640 643, in particular optionally quaternized aminoalkyl, in particular aminopropyl dimethyl polysiloxane/polyethyl oxazoline copolymers of the formula 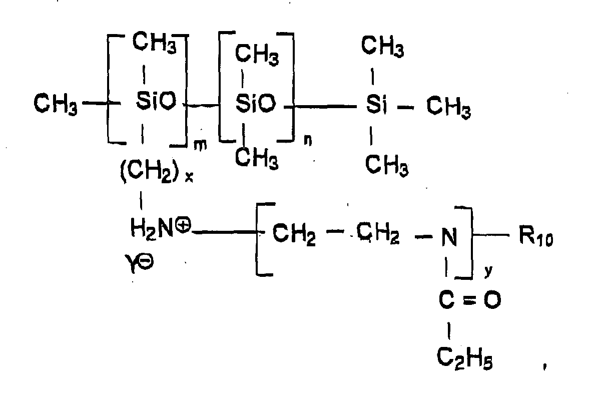 wherein m and n each are numbers from 20 to 10,000, in particular 50 to 7,000, especially 100 to 5,000, x is a number between 1 and 5, preferably 3, and y is a number from 5 to 30, R₁₀ is a C₁-C₁₂-alkyl or aryl group, in particular a methyl, ethyl or benzyl group, and Y- is an anion.

Especially suited are the organopolysiloxanes disclosed under the terms A-1, A-2 and A-3 on pages 12 to 13 of EP-A 640 643, The proportion of graft copolymers in the hair colouring compositions according to the invention ranges from 0.05% to 5%, preferably 0.1% to 2.5%, in particular 0.5% to 1.5% by weight, calculated to the total composition.

Dyeing compositions according to the present invention can contain organic solvent and also as a solubilzers, Examples of such organic solvents are benzyloxy ethanol, benzyl alcohol, phenoxy ethanol, phenoxy isopropanol, methyl phenoxy ethanol, benzyl glycerol, N-benzyl formide, N-methyl pyrrolidone, N-ethyl pyrrolidone, cinnamyl alcohol, phenethyl alcohol, p-methyl benzyl alcohol, butyl cellosolve, methyl carbitol, ethyl carbitol, propyl carbitol, butyl carbitol, diethyleneglycol, diethyl ether and dipropyleneglycol diethyl ether. Typically the concentration of those solvents can be in the range from 0.5% to 10%, preferably 0.5 - 5% by weight calculated to the total composition.

The hair dyeing compositions according to the invention preferably contain thickening agents. These are, for example, the various cellulose derivatives such as hydroxyalkyl celluloses, e.g., hydroxyethyl cellulose, hydroxypropyl cellulose, hydroxypropyl methyl cellulose, natural polysaccharides such as xanthan gum; guar gum and the alkoxylation products thereof in amounts from 0.1 - 5 %, preferably 0.1 - 3% and most preferably 0.1 - 2% by weight calculated to the total composition and depending on the desired consistency thereof.

Optionally, the composition of this invention can comprise further hair conditioning agents such as silicone oils either volatile or non-volatile, natural and synthetic oils. Among silicone oils those can be added to the colouring composition include dimethicone, dimethiconol, polydimethylsiloxane, DC fluid ranges from Dow Coming, natural oils such as olive oil, almond oil, avocado oil, weizenkeim oil, ricinus oil and the synthetic oils, such as mineral oil, isopropyl myristate, palmitate, stearate and isostearate, oleyl oleate, Isocetyl stearate, hexyl laurate, dibutyl adipate, dioctyl-adipate, myristyl myristate and oleyl erucate.

Another preferred compound in the composition of present invention especially in bleaching and/or highlighting composition and in dyeing composition Is ceramide type of compounds according to general formula 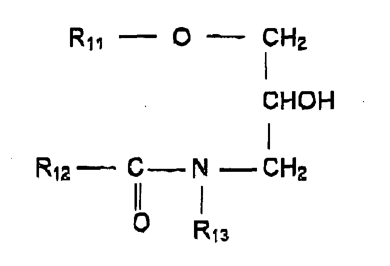 where R₁₁ and R₁₂ are independent from each other alkyl- or, alkenyl group with 10 to 22 carbon atoms, R₁₃ is alkyl or hydroxyl alkyl with 1 to 4 carbon atoms group and n is a number between 1 to 6, preferably 2 or 3. Preferred compound according to the above chemical structure is cetyl-PG-hydroxyethylpalmitamide. Concentration of ceramide type of compounds ranges from 0.01 to 2%, preferably 0.01 to 1% yb weight calculated to total composition before mixing.

The compositions of the present invention can comprise of at least one ubiquinone of the formula (I) 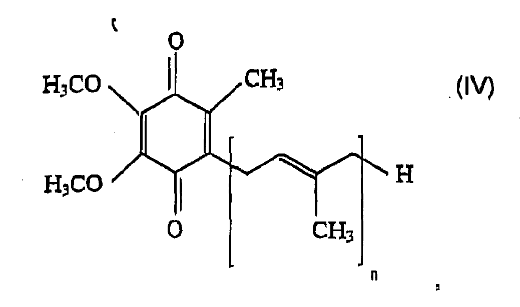 wherein n is a number from 1 to 10, Concentration of ubichinone can vary between 0.001 % and 10 % by weight, calculated to the total composition before mixing.

Dyeing composition may as well comprise UV filters of oil soluble, non-ionic, ones and/or as well those of water soluble and mainly of anionic character. Non-limiting examples are Benzophenone-1 Benzophenone-2, Benzophenone-3, Benzophenone-7, Benzophenons-6, Benzophenone-8, octylmethoxy cinnamate, homosalat to those of oil soluble ones and Benzophenone-4, benzophenone-9 to those anionic water soluble ones. It should be noted that the other UV filters of oil and water soluble ones should as well be possible to combine.

The mixing ratio of the bleaching and/or highlighting composition to dyeing composition, depending on the colour result to be achieved varies between 5:1 to 1:5, preferably 3:1 to 1:5 and more preferably 2:1 to 1:3, all by weight, which may further mixed with an oxidizing lotion at a weight ratio of preferably 1:1.

Among many different possibilities of application, one way of achieving bleaching and colouring effect in a single process that the bleaching and/or highlighting composition, dyeing composition and oxidizing lotion are mixed in a ratio given above and applied onto hair and left on the hair for 5 to 45 min and rinsed of from hair and shampooed if necessary. During the processing time on hair heat may as well be applied which should not exceed 45°C. Heat application may result in shortening of the processing time.

Although the preferred process for dyeing and bleaching is a single step process, two-step processes may as well be used with the compositions of the present Invention. One of the ways of carrying out in two steps Is that in the first step, bleaching and/or highlighting composition of the present invention is firstly mixed with an oxidizing agent and applied onto hair and left 5 to 20 min and afterwards without rinsing off the dyeing composition is applied onto hair and additionally processed for 5 to 30 min and rinsed off from hair and shampooed, if necessary. During the processing time in both steps on the hair heat can as well be applied which should not exceed 45°C. Heat application may result in shortening of the processing time.

In another way of carrying out the Invention in two step is that in the first step, bleaching and/or highlighting composition of the present invention is firstly mixed with an oxidizing agent and applied onto hair and left 5 to 45 min and afterwards rinsed off from hair and subsequently in the second step dyeing composition is applied onto hair and additionally processed for 5 to 45 min and rinsed off from hair and shampooed, if necessary. During the processing time in both steps on the hair heat can as well be applied which should not exceed 45°C. Heat application may result in shortening of the processing time.

The composition of the present invention can contain additional Ingredients such as preservatives, chelating agents, fragrance and substances customarily used in cosmetic bleaching and colouring compositions of keratin fibres, especially hair.

The invention is illustrated with the following examples, but not limited to.

### Example 1

### Bleaching / Highlighting composition in suspension form

| | |
|---|---|
| Hydroxyethylcellulose | 1,40% by weight |
| Cellulose gum | 3.20 |
| Xanthan gum | 0.30 |
| Tetrasodium EDTA | 2.00 |
| Sodium carbonate | 1.00 |
| Ammonium persulfate | 14.00 |
| Potassium persulfate | 36.60 |
| Sodium metasilicate | 10.20 |
| Corn starch | 1.10 |
| Diatomaceous Earth | 1.10 |
| Polyquaternium -10 | 0.10 |
| Di(ethylhexyl)carbonate | 29.00 |

The above composition is prepared as disclosed in the description.

Bleaching effect of the above composition was evaluated to be excellent. In addition hair was found to be less damaged (natural feeling upon touching), well combable, having good elasticity and shine,

In addition the above composition was compared to the one in powder form not comprising any diethylhexylcarbonate in a half side test and the preference of the hair dressers in 70% of the cases on the side bleached with the composition of the present invention. In the remaining 30% of the cases comparable (no or minor difference) results were obtained.

In all tests carried out with Example 1 or with comparative composition the oxidizing lotion with the composition below was used, 1 part of bleaching composition was mixed with 2 parts of oxidizing lotion,

### Oxidizing Lotion

| | |
|---|---|
| Hydrogen peroxide | 9.00 (% by wt.) |
| Cetyl stearyl alcohol | 1.70 |
| Phosphoric acid | 0.50 |
| Sodium lauryl sulfate | 0.20 |
| Coenzyme Q10 | 0.05 |
| Cetyl PG hydroxyethyl palmitamide | 0.10 |
| Disodium hydrogen phosphate | 0.10 |
| Salicylic acid | 0.10 |
| Water | ad 100.00 |

### Example 2

Bleaching and colouring in a single process was carried our using the bleaching composition of example 1, dyeing composition according to the composition below and an oxidizing lotion composition of example 1, however with 6% hydrogen peroxide content instead of 9%.

### Dyeing composition

| | % by weight |
|---|---|
| | I |
| Cocamide MEA | 4.00 |
| Cetearyl alcohol | 10.00 |
| Tegin P | 1.40 |
| Propylene Glycol | 2.40 |
| Oleic acid | 3.00 |
| Coenzyme Q10 | 0.10 |
| Ammonium chloride | 0.50 |
| Tetrasodium EDTA | 0.20 |
| Sodium lauryl sulfate | 1.50 |
| Organopolysiloxane A1 of EP 640 643 | 0.20 |
| Cetyl PG hydroxyethyl palmitamide | 0.10 |
| Basic red 51 | 0,50 |
| Water | to 100 |

The bleaching composition of example 1, dyeing composition of above and oxidizing lotion were mixed at a weight ratio of 1:1:1 and was applied onto parts of hair (streak) and left 30 min at 40°C and rinsed off with water and shampooed. Intensive highlighted red streaks were obtained.

### Example 3

Bleaching and colouring in a single process was carried our using the bleaching composition of example 1, dyeing composition of example 2 which additionally comprised 0.5% acid red 52 (the amount of water was reduced) and an oxidizing lotion composition of example 1, however with 6% hydrogen peroxide content instead of 9%, by weight.

The bleaching composition of example 1, dyeing composition of example 2 with additional 0.5% by weight Acid red 52 content and oxidizing lotion were mixed at a weight ratio of 1:1:1 and was applied onto parts of hair (streak) and left 30 min at 40°C and rinsed off with water and shampooed. Intensive highlighted red streaks were obtained.

## Claims

1. Water free composition for bleaching and/or highlighting keratin fibres especially human hair based on at least one compound with bleaching and/or highlighting effect **characterized in that** it comprises at least one dialkyl carbonate of general formula
R₁ O C(O) O R₂
where R₁ and R₂ are independent from each other linear or branched saturated alkyl chains with 6 to 22 C atoms.

2. Composition according to claim 1 **characterised in that** dialkyl carbonate is selected from di(caprylyl) carbonate and di(ethylhexyl) carbonate.

3. Composition according to claims 1 and 2 **characterised in that** it comprises at least one dialkyl carbonate at a concentration of 3 to 50% by weight calculated to total composition.

4. Composition according to any of the preceding claims **characterised in that** it comprises at least one compound with bleaching and/or highlighting effect at a concentration of 5 to 85% by weight calculated to total composition.

5. Composition according to any of the preceding claims **characterised in that** it comprises additional lipophilic compounds.

6. Composition according to any of the preceding claims **characterised in that** it comprises polymer,

7. Composition according to claim 6 **characterised in that** it comprises cationic polymer.

8. Composition according to any of the preceding claims **characterised in that** it is a suspension.

9. Composition according to any of the preceding claims **characterised in that** it comprises at least one direct dye.

10. Composition according to any of the preceding claims **characterised in that** It comprises ceramide type of compound.

11. Composition according to any of the preceding claims **characterised in that** it comprises ubichinone.

12. Use of a composition according to any of the preceding claims for bleaching and/or highlighting hair.

13. Process for bleaching and/or highlighting hair **characterised in that** a composition according to claims 1 to 11 is mixed with a composition comprising at least one oxidizing agent and applied onto hair and after processing 5 to 45 min rinsed of from hair and hair is optionally shampooed.

14. Process according to claim 11 **characterised in that** the composition applied onto hair after mixing of a composition according to claims 1 to 11 with a composition comprising at least one oxidizing agent has a pH between 8 to 11.5.

15. Process for producing a composition according to claims 1 to 11 **characterised in that** powder components of the composition is mixed first in a suitable mixer and subsequently at least one dialkyl carbonate of the general formula
R₁ O C(O) O R₂
where R₁ and R₂ are independent from each other linear or branched saturated alkyl chains with 6 to 22 C atoms is added portion wise.
